# FASCICULE DE BREVET EUROPEEN

(11) **EP 0 819 421 B1**
(45) Date de publication et mention de la délivrance du brevet: **06.10.1999**
(21) Numéro de dépôt: 97115409.1
(22) Date de dépôt: 11.10.1996
(51) Int. Cl.: A61K 7/09, A61K 7/06

(54) **Utilisation de la monoéthanolamine pour préparer une composition destinée à déterminer les cuirs chevelus sensibles**
Verwendung des Monoethanolamins zur Herstellung einer Zusammensetzung zur Ermittlung von empfindlicher Kopfhaut
Use of monoethanolamin for the preparation of a composition used to determinate the sensible scalp

(30) Priorité: 26.10.1995 FR 9512654
(43) Date de publication de la demande: 21.01.1998
(62) Demande divisionnaire de: 96402171.1
(73) Titulaire: L'OREAL, 75008 Paris (FR)
(72) Inventeur: De Lacharriere, Olivier, 75015 Paris (FR); Loussouarn, Geneviévre, 92110 Clichy (FR); Breton, Lionel, 78000 Versailles (FR)
(74) Mandataire: Miszputen, Laurent

(56) Documents cités:
- EP-A- 0 680 749
- DE-A- 3 535 351
- FR-A- 2 707 486

## Description

La présente invention se rapporte à l'utilisation de la monoéthanolamine pour préparer une composition destinée à déterminer les cuirs chevelus sensibles et à un procédé pour déterminer les cuirs chevelus sensibles.

On sait que la technique la plus usuelle pour obtenir une déformation permanente des cheveux, appelée ci-après 〈〈 permanente 〉〉, consiste, dans un premier temps, à réaliser l'ouverture des liaisons disulfures -S-S- de la kératine (cystine) à l'aide d'une composition contenant un agent réducteur (étape de réduction) puis, après avoir rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action de l'eau ou aux lavages avec un shampooing, et ceci par opposition aux simples techniques classiques de déformation temporaire, telles que de mise en pli.

Les compositions réductrices utilisables pour la mise en oeuvre de la première étape d'une opération de permanente contiennent généralement, à titre d'agents réducteurs, des sulfites, des bisulfites, des alkyl-phosphines ou de préférence des thiols. Parmi ces derniers, ceux couramment utilisés sont la cystéïne et ses dérivés, la cystéamine et ses dérivés, l'acide thiolactique et ses esters, l'acide thioglycolique ainsi que ses esters, notamment le monothioglycolate de glycérol.

Concernant les compositions oxydantes nécessaires à la mise en oeuvre de l'étape de fixation, on fait le plus souvent appel, dans la pratique, à des compositions à base d'eau oxygénée ou de bromate alcalin.

Certains agents réducteurs et oxydants, ainsi que certains adjuvants utilisés pour les permanentes peuvent entraîner des irritations et/ou des sensations dinconfort (par exemple fourmillements, èchauffements, démangeaisons), et cet effet irritant est ressenti en particulier chez certaines personnes présentant un cuir chevelu sensible.

En effet, certains sujets présentent une réactivité du cuir chevelu plus importante que d'autres. En particulier, ils répondent à certains produits tels que les colorants capillaires, les permanentes, les tensioactifs. Les individus, outre les signes cliniques mentionnés ci-dessus, ont la particularité d'avoir une tendance à répondre plus fortement à certains tests de provocation qui visent à reproduire ces signes cliniques. Parmi ces tests, la demanderesse a mis au point le test à la capsaïcine et celui à la monoéthanolamine.

Le test à la capsaïcine est décrit dans la demande de brevet français n° 94-05537 déposée par la demanderesse.

La présente invention a pour objet l'utilisation de la monoéthanolamine en solution aqueuse pour déterminer les cuirs chevelus sensibles.

Le test à la monoéthanolamine consiste à préparer une solution aqueuse contenant 10 % de monoéthanolamine, à verser sur un coton 3 ml de cette solution, à appliquer ce coton 10 fois sur les zones testées du cuir chevelu et à évaluer les signes cliniques qui apparaissent à 30 secondes, 2 minutes, 5 minutes, 10 minutes et 15 minutes après application. Ces signes sont essentiellement des signes subjectifs (picotements, fourmillements, démangeaisons, échauffements), éventuellement associés à un érythème.

Aussi, a présente invention a encore pour objet un procédé pour déterminer les cuirs chevelus sensibles, consistant à appliquer sur le cuir chevelu une solution aqueuse de monoéthanolamine et à évaluer les signes cliniques qui apparaissent.

En outre, la Demanderesse a découvert que l'utilisation d'antagonistes de substance P et/ou d'antagonistes de CGRP permettait d'obtenir un effet préventif et/ou curatif de l'irritation due aux composés utilisés dans les compositions de permanente des personnes à cuir chevelu sensible.

La substance P est un élément chimique polypeptidique élaboré et libéré par les terminaisons nerveuses. Elle fait partie de la famille des tachykinines. La substance P intervient notamment dans la transmission de la douleur, dans des maladies du système nerveux central, telles que l'anxiété et la schizophrénie, dans des maladies respiratoires et inflammatoires, dans des maladies gastro-intestinales, dans des maladies rhumatismales et dans certaines maladies dermatologiques telles que l'eczéma.

Le CGRP (peptide dérivé du gène de la calcitonine : Calcitonin Gene Related Peptide en langue anglosaxonne) est un élément chimique polypeptidique élaboré et libéré par une terminaison nerveuse. Le CGRP intervient notamment dans des maladies respiratoires et inflammatoires, dans des maladies allergiques et dans certaines maladies dermatologiques telles que l'eczéma ou le prurigo.

Personne jusqu'à ce jour n'avait envisagé d'utiliser des antagonistes de substance P ou de CGRP en association avec des composés utilisés dans les permanentes et notamment les matières réductrices et/ou oxydantes, en vue d'éliminer l'effet irritant et/ou d'inconfort provoqués par ces composés chez les personnes à cuir chevelu sensible.

Pour obtenir uneffet préventif et/ou curatif de l'irratation, on utilise une composition notamment cosmétique pour le cuir chevelu contenant, dans un milieu physiologiquement acceptable au moins un antagoniste choisi parmi les antagonistes de substance P et les antagonistes de CGRP et au moins un actif à effet secondaire irritant, qui est un agent réducteur et/ou un agent oxydant à l'exception des peroxydes et des crésols.

La composition est notamment une composition pour la déformation permanente des fibres kératiniques, et en particulier une composition pour la déformation permanente des cheveux humains.

L'antagoniste de substance P ou de CGRP peut être appliqué sur les fibres kératiniques avant et/ou pendant et/ou après les étapes de réduction et/ou d'oxydation.

Le procédé de traitement des fibres kératiniques, en particulier des cheveux des cuirs chevelus sensibles, en vue d'obtenir une déformation permanente de ces fibres, comprend les étapes suivantes :
i) application sur les fibres kératiniques d'une composition réductrice, les moyens nécessaires à la mise sous tension mécanique des fibres kératiniques étant mis en oeuvre avant, pendant ou après cette application de la composition réductrice,
ii) après action de la composition réductrice, rinçage des fibres kératiniques,
iii) application sur les fibres kératiniques d'une composition oxydante,
iv) séparation des moyens de mise sous tension avant ou après l'étape iii,
v) rinçage éventuel des fibres kératiniques,
vi) application sur les fibres kératiniques d'une composition contenant au moins un antagoniste choisi parmi les antagonistes de substance P et les antagonistes de CGRP, au cours de l'une au moins des étapes i à v et/ou après l'une au moins de ces étapes i à v.

Selon un mode particulier de réalisation, les différentes compositions sont conditionnées séparément sous forme d'un kit, selon un agencement bien connu de l'homme du métier.

Le kit pour le traitement des fibres kératiniques, en particulier des cheveux des cuirs chevelus sensibles, en vue d'obtenir une déformation permanente de ces fibres, comprend une première composition contenant un agent réducteur et une seconde composition contenant un agent oxydant, les deux compositions, destinées à être appliquées l'une après l'autre sur les fibres kératiniques, étant conditionnées séparément, l'une au moins de ces compositions contenant un antagoniste choisi parmi les antagonistes de substance P et les antagonistes de CGRP.

Le kit pour le traitement des fibres kératiniques, en particulier des cheveux, en vue d'obtenir une déformation permanente de ces fibres, peut comprendre aussi une première composition contenant un agent réducteur, une seconde composition contenant un agent oxydant, une troisième composition contenant un antagoniste choisi parmi les antagonistes de substance P et les antagonistes de CGRP, les trois compositions, destinées à être appliquées les unes après les autres sur les fibres kératiniques, étant conditionnées séparément.

Pour qu'une substance soit reconnue comme un antagoniste de substance P, elle doit répondre à la caractéristique suivante :
- avoir une activité pharmacologique antagoniste de la substance P, c'est-à-dire induire une réponse pharmacologique cohérente dans au moins l'un des deux tests suivants :
   - la substance antagoniste doit diminuer l'extravasation du plasma au travers de la paroi vasculaire induite par la capsaïcine ou par une stimulation nerveuse antidromique, ou bien
   - la substance antagoniste doit provoquer une inhibition de la contraction des muscles lisses induites par l'administration de substance P.

L'antagoniste de substance P peut en outre avoir une affinité sélective pour les récepteurs NK1 des tachykinines.

L'antagoniste de substance P peut être fonctionne ou réceptoriel, c'est-à-dire inhiber la synthèse et/ou la libération de substance P, ou empêcher sa fixation et/ou moduler son action. Il peut être choisi parmi les composés connus comme antagonistes de substance P, notamment les peptides, les dérivés non peptidiques, et plus précisément ceux comportant un hétèrocycle azoté, soufré ou oxygéné, ou les composés azotés comportant un atome d'azote lié directement ou indirectement à un cycle benzénique. Il peut être choisi également parmi les sels de métaux monovalents, divalents ou trivalents, et parmi des extraits d'origine végétale et/ou bactérienne.

Ainsi, on peut utiliser par exemple comme peptide antagoniste de substance P le sendide et le spantide II. On peut également utiliser les peptides décrits dans les documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes de substance P non peptidiques sont notamment des composés hétérocycliques, notamment azotés, soufrés ou oxygénés, ou des composés comprenant un atome d'azote lié directement ou indirectement à un ou plusieurs cycles benzéniques.

Comme composé hétérocyclique, on peut utiliser ceux comportant un hétérocycle azoté, décrits dans les documents suivants : EP-A-360390, EP-A-429366, EP-A-430771, EP-A-499313, EP-A-514273, EP-A-514274, EP-A-514275, EP-A-514276, EP-A-520555, EP-A- 528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, W0-A-93/01170, WO-A-93/06099, WO-A-93/09116. En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle ou un dérivé d'isoindole.

Comme autres composés hétérocycliques, on peut citer les composés hétérocycliques oxygénés ou soufrés tels que les dérivés du furanne, les dérivés du benzofuranne, les dérivés du thiophène et les dérivés du benzothiophène, comportant éventuellement des substituants azotés, tels que les composés hétérocycliques décrits dans les documents US-A-4931459, US-A-4910317 et EP-A-299457, et plus spécialement les alcoxy- et/ou aryloxy- tétrazolylbenzofuranne-carboxamides ou les alcoxy- et/ou aryloxy- tétrazolylbenzothiophène-carboxamides.

Comme composés comportant un atome d'azote lié directement ou indirectement à un noyau benzénique, on peut citer ceux décrits dans les documents suivants : EP-A-522808, WO-A-93/01165 et WO-A-93/10073. On peut citer notamment les dérivés d'éthylène diamine, tels que la N,N'-bis-di-(3,5-dimethylbenzyle)- éthylène diamine, la N,N'-bis-di-(3,5-dimethoxybenzyle)-éthylène diamine ; ces composés sont décrits comme intermédiaires de synthèse dans le document WO-A-93/11338 déposé au nom de la demanderesse.

Les sels de métaux monovalents, divalents ou trivalents, utilisables comme antagonistes de substance P, peuvent être des sels de cobalt ; des sels des éléments de la colonne II A de la classification périodique, notamment des sels de béryllium, de magnésium ou de métal alcalino-terreux, en particulier de strontium, de calcium et de baryum ; des sels de lanthanides, notamment de lanthane et de gadolinium ; des sels d'yttrium ; des sels de zinc ; des sels de manganèse ; des sels de cuivre ; des sels de rubidium ; des sels de lithium.

Ces sels peuvent être par exemple des chlorures, des carbonates, des bicarbonates, des borates, des nitrates, des acétates, des hydroxydes, des sulfates, des persulfates, des glycérophosphates, ainsi que des sels d'α-hydroxyacides ou des sels d'acides de fruits (citrate, tartrate, lactate, malate), ou encore des sels d'acides aminés (aspartate, arginate, glucocholate, fumarate) ou des sels d'acides gras (palmitate, oléate, caséinate, béhénate).

De façon avantageuse, le sel est un sel de strontium, et notamment le chlorure de strontium ou le nitrate de strontium.

Les extraits d'origine bactérienne peuvent être des extraits d'au moins une bactérie filamenteuse non photosynthétique.

Comme extrait d'origine végétale, on peut citer notamment ceux provenant d'*Iris germanica*, d'*Iris florentina*, d'*Iris pallida*, de *Crocus versicolor*, de *Romulea bulbucodium* ou encore de *Gladiolus communis*. Plus particulièrement, on utilise un extrait végétal issu d'une iridacée et préférentiellement du matériel végétal d'*Iris pallida*. Toute méthode d'extraction connue de l'homme du métier peut être utilisée pour préparer l'extrait contenu dans la composition. On peut, en particulier, citer les extraits alcooliques, notamment éthanoliques, ou encore hydroalcooliques. On peut également utiliser un extrait préparé par la méthode décrite dans la demande de brevet français n° 95-02379 déposée par la demanderesse.

Pour qu'une substance soit reconnue comme un antagoniste de CGRP, elle doit répondre notamment aux caractéristiques suivantes :
- avoir une affinité pour les récepteurs au CGRP et/ou
- avoir une activité pharmacologique antagoniste du CGRP, c'est-à-dire induire une réponse pharmacologique cohérente notamment dans l'un des tests suivants :
   - la substance antagoniste doit diminuer la vasodilatation induite par la capsaïcine et/ou
   - la substance antagoniste doit provoquer une inhibition de la libération de CGRP par les fibres nerveuses sensitives et/ou
   - la substance antagoniste doit provoquer une inhibition de la contraction du muscle lisse du canal déférent induite par le CGRP.

On peut utiliser par exemple comme antagoniste de CGRP, le CGRP 8-37 (séquence des acides aminés 8 à 37 de la partie terminale du CGRP), un anticorps anti-CGRP.

Dans les compositions pour cuirs chevelus sensibles, l'antagoniste de substance P ou de CGRP est utilisé de préférence en une quantité allant de 0,000001 à 30 % en poids par rapport au poids total de la composition, et en particulier en une quantité allant de 0,0001 à 10 % en poids par rapport au poids total de la composition.

L'antagoniste de substance P ou de CGRP joue essentiellement un rôle sur les agents réducteurs et/ou oxydants ayant un effet irritant, les agents non irritants étant par ailleurs présents dans la composition pour permettre d'atteindre la déformation souhaitée.

Dans la première étape (i) du procédé de traitement, on applique sur les fibres kératiniques une composition réductrice contenant au moins un agent actif approprié pour la réduction des liaisons disulfures de la kératine. Cette application peut être réalisée avant, pendant ou après l'habituelle étape de mise sous tension des fibres kératiniques. L'antagoniste de substance P ou de CGRP peut être présent dans la composition réductrice ou dans une composition appliquée sur les fibres kératiniques avant ou après l'étape de réduction.

L'habituelle étape de mise sous tension des fibres kératiniques, notamment des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis et analogues.

Parmi les agents actifs appropriés pour la réduction des liaisons disulfures de la kératine, on peut citer les sulfites, les bisulfites, les alkyl-phosphines ou, de préférence, les thiols. Parmi ces derniers, ceux préférentiellement utilisés sont choisis parmi l'acide thioglycolique, le monothioglycolate de glycérol ou de glycol, la cystéamine et ses dérivés acylés en C₁-C₄ tels que la N-acétyl cystéamine ou la N-propionyl cystéamine, la cystéine, la N-acétylcystéine, les esters de cystéine, tels que le cystéïnate de glycérol, les N-mercaptoalkylamides de sucres tels que le N-(mercapto-2-éthyl)gluconamide, l'acide thiolactique et ses esters tels que le monothiolactate de glycérol, l'acide 3-mercaptopropionique et ses esters tels que le 3-mercaptopropionate de glycérol, l'acide thiomalique, l'acide 2-hydroxy 3-mercaptopropionique et ses esters, tels que le 2-hydroxy 3-mercaptopropionate de glycérol, la pantéthéine, le thioglycérol, les sulfites ou les bisulfltes d'un métal alcalin ou alcalino-terreux, les N-(mercaptoalkyl)ω-hydroxyalkylamides décrits dans la demande de brevet EP-A-354835 et les N-mono- ou N,N-dialkylmercapto 4-butyramides décrits dans la demande de brevet EP-A-368763, les aminomercaptoalkylamides décrits dans la demande de brevet EP-A-432000, les dérivés des acides N-(mercaptoalkyl) succinamiques ou des N-(mercaptoalkyl) succiminides décrits dans la demande de brevet EP-A-465342, les alkylaminomercaptoalkylamides décrits dans la demande de brevet EP-A-514282, le mélange de thioglycolate d'hydroxy-2-propyle et de thioglycolate d'hydroxy-2-méthyl-1 éthyle décrit dans la demande de brevet FR-A-2679448, les N-mercaptoalkyl alcanediamides décrits dans la demande de brevet EP-A-653202.

On utilise de préférence l'acide thioglycolique, l'acide thiolactique, la cystéïne et ses dérivés, la cystéamine et ses dérivés, l'acide 3-mercaptopropionique, ainsi que leurs esters ou leurs sels, notamment le monothioglycolate de glycérol.

Ces agents actifs peuvent être utilisés seuls ou en mélange.

Lorsque l'on utilise l'acide thioglycolique, l'acide thiolactique, l'acide 3-mercaptopropionique, l'acide 2-hydroxy 3-mercaptopropionique, la cystéine ou la cystéamine, ou l'un de leurs sels ou de leurs dérivés, à titre d'agent réducteur, le pH de l'ensemble de la composition est de préférence compris entre 6 et 11,5 et encore plus préférentiellement entre 7 et 10.

Lorsque l'on utilise les esters de l'acide thioglycolique ou de l'acide thiolactique ou de l'acide 3-mercaptopropionique, de la cystéïne ou de l'acide 2-hydroxy 3-mercaptopropionique, à titre d'agent réducteur, le pH de l'ensemble de la composition est de préférence compris entre 5 et 10 et encore plus préférentiellement entre 6 et 9.

Les agents réducteurs mentionnés ci-avant sont généralement présents à une concentration qui peut aller de 1 à 20 % en poids par rapport au poids total de la compositon réductrice.

Les pH des compositions réductrices peuvent être ajustés classiquement par ajout d'agents basifiants en vue de rendre plus efficaces les agents réducteurs. Ces agents basifiants peuvent être choisis par exemple parmi la soude, l'ammoniaque, la monoéthanolamine, la diéthanolamine, la triéthanolamine, l'isopropanolamine, la propanediamine-1,3, un carbonate ou bicarbonate de métal alcalin ou d'ammonium, un carbonate ou bicarbonate d'amines primaires, secondaires ou tertiaires, ou un carbonate organique tel que le carbonate de guanidine, tous ses composés pouvant bien entendu être pris seuls ou en mélange.

L'antagoniste de substance P ou de CGRP permet en outre de réduire l'effet irritant de certains de ces agents basifiants.

La composition réductrice peut se présenter sous la forme d'une lotion, épaisse ou non, d'une crème, d'un gel ou de toute autre forme appropriée et peut contenir des additifs connus pour leur utilisation dans les compositions réductrices pour la déformation permanente des cheveux.

La composition réductrice peut être également du type exothermique.

La composition réductrice peut également contenir un solvant tel que par exemple l'éthanol, le propanol, l'isopropanol ou encore le glycérol à une concentration maximale de 20 % par rapport au poids total de la composition.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc.

On peut également utiliser des liquides ou des gels contenant des agents épaississants tels que des polymères ou des copolymères carboxyvinyliques qui "collent" les cheveux et les maintiennent dans la position lisse pendant le temps de pose.

Enfin, les compositions peuvent être également sous forme dite "auto-neutralisante" ou encore "auto-régulée" et dans ce cas, les agents réducteurs sont associés à au moins un disulfure connu pour son utilisation dans une composition réductrice pour permanente auto-neutralisante.

Parmi de tels disulfures connus, on peut notamment mentionner l'acide dithioglycolique, le dithioglycérol, la cystamine, la N,N'-diacétyl-cystamine, la cystine, la pantéthine, et les disulfures des N-(mercapto-alkyl) ω-hydroxyalkylamides décrits dans la demande de brevet EP-A-354835, les disulfures des N-mono ou N,N-dialkylmercapto-4 butyramides décrits dans la demande de brevet EP-A-368763, les disulfures des aminomercapto-alkylamides décrits dans la demande de brevet EP-A-432000, les disulfures des dérivés des acides N-(mercaptoalkyl)-succinamiques ou des N-(mercaptoalkyl)-succinimides décrits dans la demande de brevet EP-A-465342, les disulfures de alkylaminomercaptoalkylamides décrits dans la demande de brevet EP-A-514282 et les disulfures des N-mercaptoalkyl alcanediamides décrits dans la demande de brevet EP-A-653202. Ces disulfures sont généralement présents dans un rapport molaire de 0,5 à 2,5, et de préférence de 1 à 2, par rapport à l'agent réducteur.

Avant de procéder à l'étape suivante de rinçage (ii), il convient, de manière classique, de laisser reposer pendant quelques minutes, généralement entre 2 et 40 minutes, de préférence entre 5 et 30 minutes, les fibres kératiniques sur laquelle a été appliquée la composition réductrice, et ceci de façon à bien laisser le temps au réducteur d'agir correctement sur les fibres kératiniques. Cette phase d'attente est effectuée généralement en laissant reposer à l'air libre (température ambiante) les fibres kératiniques traitées, mais elle peut être également effectuée à une température plus élevée. Pendant cette phase d'attente, on prend soin que les fibres kératiniques ne sèchent pas complètement et restent humides jusqu'au moment de la mise en oeuvre de l'étape suivante.

Dans la deuxième étape du procédé (ii), les fibres kératiniques imprégnées de la composition réductrice sont donc rincées soigneusement par une composition aqueuse, éventuellement additionnée d'un antagoniste de substance P et/ou d'un antagoniste de CGRP.

Puis, dans une troisième étape (iii), on applique sur les fibres kératiniques ainsi rincées une composition oxydante dans le but de fixer la nouvelle forme imposée aux fibres kératiniques, cette composition oxydante pouvant contenir un antagoniste de substance P et/ou un antagoniste de CGRP.

On peut retirer des fibres kératiniques les moyens mécaniques (rouleaux, bigoudis et analogues) qui maintenaient sous tension et dans la forme désirée les fibres kératiniques tout au long du traitement avant ou après l'étape de fixation.

La composition oxydante contient un agent oxydant qui peut être choisi parmi l'eau oxygénèe, un bromate alcalin, un persel, un chlorite ou un polythionate ou leur mélange, tel qu'un mélange de bromate alcalin et d'un persel. On peut utiliser par exemple le bromate de potassium, le perborate de sodium, le chlorite de sodium.

La concentration en eau oxygénée peut varier de 1 à 10 volumes, mais est de préférence de 8 volumes ; la concentration en bromate alcalin est généralement de 1 à 12 % et celle en persel de 0,1 à 15 % en poids par rapport au poids total de la composition oxydante.

Le pH de la composition oxydante est généralement compris entre 2 et 10.

La composition oxydante peut contenir des additifs cosmétiques bien connus pour ce type de composition.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

Le véhicule des compositions réductrice et oxydante utilisées est de préférence l'eau ou une solution hydroalcoolique d'un alcool inférieur tel que l'éthanol, l'isopropanol ou le butanol.

L'eau oxygénée peut être stabilisée par exemple par la phénacétine, l'acétanilide, les phosphates mono et trisodiques ou par le sulfate d'hydroxy-8 quinoléine.

La composition oxydante peut également contenir des agents alcalinisants ou acidifiants, des agents conservateurs, des agents séquestrants, des opacifiants.

Enfin, les fibres kératiniques imprégnées de la composition oxydante sont rincées soigneusement, généralement à l'eau éventuellement additionnée d'un antagoniste de substance P et/ou d'un antagoniste de CGRP.

On obtient finalement une chevelure présentant la mise en forme souhaitée, sans qu'apparaisse irritation ou inconfort.

Les quantités des différents constituants des compositions ci-dessous sont celles classiquement utilisées dans les domaines considérés.

L'exemple qui suit est destiné à illustrer l'invention sans pour autant en limiter la portée.

### Exemple 1 : Test à la monoéthanolamine

On a réalisé un test mettant en évidence l'effet apaisant d'un antagoniste de substance P après induction d'une réaction par application de monoéthanolamine sur 14 sujets ayant le cuir chevelu sensible.

Le test a consisté à traiter préalablement le cuir chevelu par demi-tête soit par une lotion hydroalcoolique (eau/éthanol : 90/10) à 5 % de chlorure de strontium soit par un placebo constitué d'une solution hydroalcoolique (eau/éthanol : 90/10), à appliquer ensuite une solution aqueuse à 10 % de monoéthanolamine et à évaluer cliniquement les sensations d'inconfort (picotements, démangeaisons, brûlures) jusqu'à 15 minutes après l'application de la solution aqueuse de monoéthanolamine.

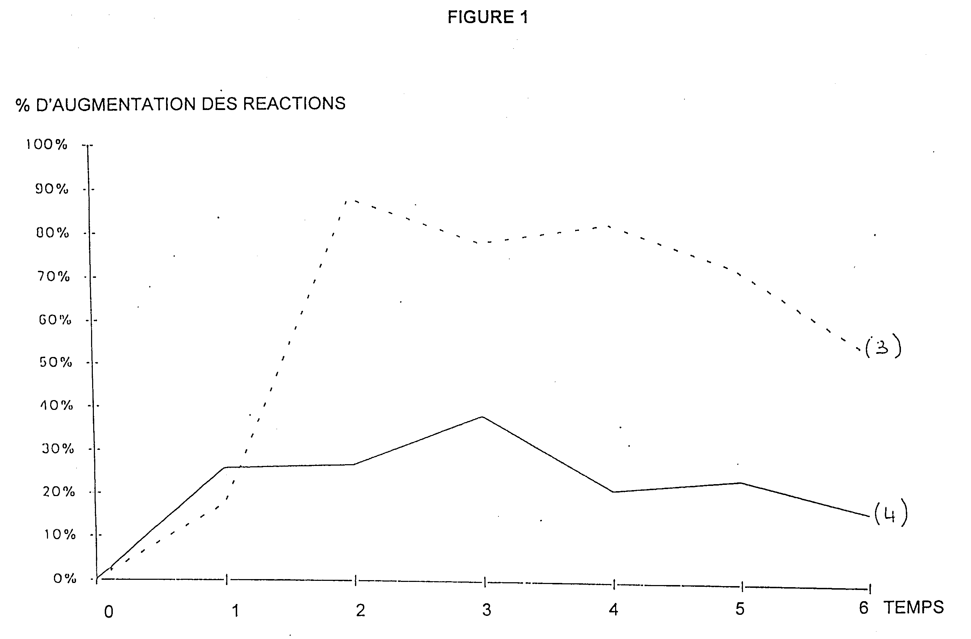

Les résultats du test sont présentés sur la figure 1 qui donne en abscisse le temps (exprimé de 0 à 6), en fonction de la moyenne du pourcentage d'augmentation des réactions en ordonnée. La courbe (3) en pointillés est celle obtenue avec le placebo et la courbe (4) en traits pleins est celle obtenue avec la lotion contenant un antagoniste de substance P.

Le temps en abscisse correspond aux temps réels suivants :
1. Après application de la lotion contenant l'antagoniste ou du placebo et avant application de la solution de monoéthanolamine ;
2. 30 secondes après application de la solution de monoéthanolamine ;
3. 2 minutes après application de la solution de monoéthanolamine ;
4. 5 minutes après application de la solution de monoéthanolamine;
5. 10 minutes après application de la solution de monoéthanolamine;
6. 15 minutes après application de la solution de monoéthanolamine;

La figure 1 met en évidence une nette diminution des réactions quand le traitement des cheveux est précédé de l'application de la lotion selon l'invention, contenant un antagoniste de substance P.

## Revendications

1. Utilisation de la monoéthanolamine en solution aqueuse pour déterminer les cuirs chevelus sensibles.

2. Utilisation selon la revendication 1, caractérisée par le fait que la monoéthanolamine est en solution aqueuse à 10 %.

3. Procédé pour déterminer les cuirs chevelus sensibles consistant à appliquer sur le cuir chevelu une solution aqueuse de monoéthanolamine et à évaluer les signes cliniques qui apparaissent.

4. Procédé selon la revendication 3, caractérisé par le fait que la monoéthanolamine est à 10 % en solution aqueuse.

5. Procédé selon la revendication 3 ou 4, caractérisé par le fait que les signes cliniques sont notés entre 30 secondes et 15 minutes après l'application.

6. Procédé selon l'une quelconque des revendications 3 à 5, caractérisé par le fait que les signes cliniques sont les picotements, les fourmillements, les démangeaisons, les brûlures et/ou les échauffements, éventuellement associés à un érythème.

## Claims

1. Use of monoethanolamine in aqueous solution for determining sensitive scalps.

2. Use according to Claim 1, characterized in that the monoethanolamine is at 10% in aqueous solution.

3. Method for determining sensitive scalps, which consists in applying, to the scalp, an aqueous monoethanolamine solution and in evaluating the clinical signs which appear.

4. Method according to Claim 3, characterized in that the monoethanolamine is at 10% in aqueous solution.

5. Method according to Claim 3 or 4, characterized in that the clinical signs are recorded between 30 seconds and 15 minutes after application.

6. Method according to any one of Claims 3 to 5, characterized in that the clinical signs are smarting, pins and needles, itching, burning sensations and/or inflammation, optionally in combination with an erythema.

## Patentansprüche

1. Verwendung von Monoethanolamin in wäßriger Lösung zur Ermittlung von empfindlicher Kopfhaut.

2. Verwendung nach Anspruch 1, dadurch gekennzeichnet, daß das Monoethanolamin in einer wäßrigen Lösung von 10 % vorliegt.

3. Verfahren zur Ermittlung von empfindlicher Kopfhaut, das darin besteht, auf die Kopfhaut eine wäßrige Lösung von Monoethanolamin aufzutragen und die auftretenden klinischen Anzeichen zu bewerten.

4. Verfahren nach Anspruch 3, dadurch gekennzeichnet, daß das Monoethanolamin in einer wäßrigen Lösung von 10 % vorliegt.

5. Verfahren nach Anspruch 3 oder 4, dadurch gekennzeichnet, daß die klinischen Anzeichen während einer Zeitspanne von 30 Sekunden bis 15 Minuten nach dem Auftragen aufgenommen werden.

6. Verfahren nach einem der Ansprüche 3 bis 5, dadurch gekennzeichnet, daß es sich bei den klinischen Anzeichen um Prickeln, Kribbeln, Jucken, Brennen und/oder Hitzegefühle handelt, die gegebenenfalls von einem Erythem begleitet sind.
